(19) Europäisches Patentamt
European Patent Office
Office européen des brevets

(11) Publication number: **0 100 051**
**B1**

# (12) EUROPEAN PATENT SPECIFICATION

(45) Date of publication of patent specification: **09.04.86**

(51) Int. Cl.$^4$: **C 07 C 103/78,** A 61 K 31/165

(21) Application number: **83107029.7**

(22) Date of filing: **18.07.83**

## (54) New derivatives of 1-alkylamine-(4(p-alkyloxy-benzamide)phenoxy)-2-propanol, having beta-sympatholytic activity, their salts, and production processes thereof.

(30) Priority: **20.07.82 IT 947482**
**28.09.82 IT 951482**

(43) Date of publication of application:
**08.02.84 Bulletin 84/06**

(45) Publication of the grant of the patent:
**09.04.86 Bulletin 86/15**

(84) Designated Contracting States:
**AT BE CH DE FR GB LI LU NL SE**

(56) References cited:
**GB-A-1 199 632**
**GB-A-1 391 444**

**The file contains technical information submitted after the application was filed and not included in this specification**

(73) Proprietor: **A. Menarini S.a.S.**
**Via dei Sette Santi 3**
**I-50131 Firenze (IT)**

(72) Inventor: **Pestellini, Vittorio**
**Via Cernaia 43**
**Firenze (IT)**
Inventor: **Ghelardoni, Mario**
**Via Lambruschini 5**
**Firenze (IT)**
Inventor: **Giannotti, Danilo**
**Via Roma 46**
**Altopascio, Lucca (IT)**
Inventor: **Giolitti, Alessandro**
**Via Fabroni 45**
**Firenze (IT)**
Inventor: **Maggi, Carlo Alberto**
**Via Michelazzi 43**
**Firenze (IT)**
Inventor: **Manzini, Stefano**
**Via Novelli 49**
**Firenze (IT)**
Inventor: **Grimaldi, Guglielmo**
**Viale Europa 185c**
**Firenze (IT)**
Inventor: **Meli, Alberto**
**Corso Italia 15**
**Firenze (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 100 051 B1

Courier Press, Leamington Spa, England.

(74) Representative: **Glawe, Delfs, Moll & Partner**
**Patentanwälte**
**Rothenbaumchaussee 58 Postfach 2570**
**D-2000 Hamburg 13 (DE)**

**Description**

The invention relates to new derivatives of 1-alkylamino-3-[4(p-alkoxybenzamido)phenoxy]-2-propanol, to pharmaceutical compositions containing said products, and to methods for producing and utilizing these 1-alkylamino-3-[4(p-alkoxybenzamido)phenoxy]-2-propanol derivatives.

The main object of this patent is to provide a group of compounds which present sympatholytic activity and can be used in the therapy of troubles induced by hyperactivity of the sympathetic nervous system.

It is well known that many derivatives of 1-alkylamino-3-phenoxy-2-propanol present beta-sympatholytic activity, but it is also known that these compounds are lacking in cardioselectivity while showing intensive liver metabolism and cardiac failure risk. Those products, as for example the 1-(p-acetamido-phenoxy)-3-isopropylamino-2-propanol (A. F. Crowther, R. Howe, L. H. Smith J. Med. Chem. *14,* 6, 511, 1971), which present $\beta_1$-cardioselectivity and poor liver metabolism, show, however, no chronotropic selectivity relative to the inotropism. On the contrary, compounds presenting chronotropic selectivity such as, for example, the 1-isopropyl-amino-3-[(2-methylindol-4-yl)oxy]-2-propanol (Swiss 469.002), the (1-tert.butylamino-ethyl)-2,5-dimethoxybenzylalcohol (Levy, J. Pharmacol. Exp. Ther. *151,* 413, 1966; Wilkenfels, Levy, Arch. Int. Pharmacodyn. Ther. *176,* 218, 1968), have either no cardioselectivity or are more active on $\beta_2$ receptors than on $\beta_1$ receptors.

GB—A—1 391 444 discloses a variety of alkanolamine derivatives of the formula

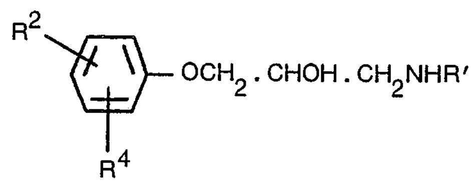

wherein $R^4$ may be hydrogen and $R^2$ may be a benzamido group substituted by one alkoxy group, and similar alkanolamine derivatives are described in US—A—3 408 387. However, no hint whatsoever can be found that the presence and the position of the alkoxy group in the benzamido group are particularly important.

It has now been found that compounds of this type having a benzamido group substituted in a specific manner present superior pharmacological properties, i.e. optimum chronoselectivity and intrinsic sympathomimetic activity combined with far superior beta blocking activity in vivo as could be evidenced by experiments.

The present invention relates to new compounds having an improved beta-sympatholytic activity, of general formula I:

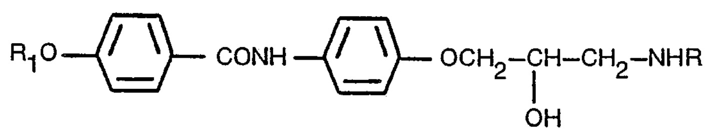

I

where $R_1$ is a linear or branched alkyl chain with 1 to 8 carbon atoms included, an alkenyl chain with 2 to 8 carbon atoms included, an aralkyl chain with 7 to 10 carbon atoms included; R is a linear or branched alkyl group with 1 to 8 carbon atoms included.

It has been found that the new compounds of general formula I and the salts thereof are medicines which present improved β-sympatholytic activity in that, besides having relevant $\beta_1$-cardioselectivity, intrinsic sympathicomimetic activity and high biodisposability, they show also a high chronotropic selectivity.

Examples of compounds according to the general formula I and included in the present invention are:

1) 1-isopropylamino-3-[4(p-methoxybenzamido)phenoxy]-2-propanol
(Form. I $R=CH(CH_3)_2$ $R_1=4—OCH_3$)
m.p. 174—176°C (Hydrochloride m.p. 208—210°C)
I.R. (nujol) ν ($cm^{-1}$): 1640 (CO)
H—NMR(DMSO) δ (p.p.m.): 1.15 (d, $2\times CH_3$), 2.7—3.0 (m, $CH_2$+CH), 3.8—4.1 (m, $CH_2$+CH), 6.9—8.3 (m, $2\times C_6H_4$)

2) 1-t-butylamino-3-[4(p-methoxybenzamido)phenoxy]-2-propanol hydrochloride
(Form. I $R=C(CH_3)_3{\cdot}HCl$ $R_1=4—OCH_3$)
m.p. 210— 212°C
I.R. (nujol) ν ($cm^{-1}$): 1650 (CO)
H—NMR($D_2O$) δ (p.p.m.): 1.30 (s, $3\times CH_3$) 2.8—3.2 (m, $CH_2$+CH) 3.5 (s, $CH_3$) 3.7—4.4 (m, $CH_2$+CH) 6.5—7.6 (m, $2\times C_6H_4$)

3) 1[4(p-ethoxybenzamido)phenoxy]-3-isopropylamino-2-propanol hydrochloride
(Form. I R=$CH(CH_3)_2 \cdot HCl$ $R_1$=4—$OC_2H_5$)
m.p. 215—217°C
I.R. (nujol) ν ($cm^{-1}$): 1640 (CO)
H—NMR(DMSO) δ (p.p.m.): 1.15 (d, 2×$CH_3$) 1.25 (t, $CH_3$) 2.8—3.6 (m, $CH_2$+CH) 3.8—4.3 (m, 2×$CH_2$+CH) 6.8—8.0 (m, 2×$C_6H_4$) 10.0 (s, NH)

4) 1[4(p-allyloxybenzamido)phenoxy]-3-isopropylamino-2-propanol hydrochloride
(Form. I R=$CH(CH_3)_2 \cdot HCl$ $R_1$=4—$OCH_2CH=CH_2$)
m.p. 200—202°C
I.R. (nujol) ν ($cm^{-1}$): 1635 (CO)

5) 1-isopropylamino-3-[4(p-propoxy-benzamido)phenoxy]-2-propanol hydrochloride
(Form. I R=$CH(CH_3)_2 \cdot HCl$ $R_1$=4—$OC_3H_7$)
m.p. 224—225°C
I.R. (nujol) ν ($cm^{-1}$): 1635 (CO)
H—NMR(DMSO) δ (p.p.m.): 0.95 (t, $CH_3$) 1.25 (d, 2×$CH_3$) 1.75 (s, $CH_2$) 2.8—3.4 (m, $CH_2$+CH) 3.7—4.4 (m, $CH_2$+CH) 4 (t, $CH_2$) 6.8—8.1 (m, 2×$C_6H_4$) 10.1 (s, NH)

6) 1-isopropylamino-3-[4(p-isopropoxybenzamido)phenoxy]-2-propanol hydrochloride
(Form. I R=$CH(CH_3)_2$ $R_1$=4—$OCH(CH_3)_2$)
m.p. 218—219°C
I.R. (nujol) ν ($cm^{-1}$): 1635 (CO)
H—NMR(DMSO) δ (p.p.m.): 1.20 (d, 4×$CH_3$) 6.8—8.0 (m, 2×$C_6H_4$) 10.0 (s, NH)

7) 1[4(p-butoxybenzamido)phenoxy]-3-isopropylamino-2-propanol
(Form. I R=$CH(CH_3)_2$ $R_1$=4—$OC_4H_9$)
m.p. 152—154°C
I.R. (nujol) ν ($cm^{-1}$): 1635 (CO)
H—NMR(DMSO) δ (p.p.m.): 1.05 (t, $CH_3$) 1.10 (d, 2×$CH_3$) 1.3—2.0 (m, 2×$CH_2$) 2.7—3.1 (m, $CH_2$+CH) 3.6—4.2 (m, $CH_2$+CH) 6.8—8.1 (m, 2×$C_6H_4$) 10.0 (s, NH)

8) 1-isopropylamino-3-[4(p-pentoxybenzamido)phenoxy]-2-propanol
(Form. I R=$CH(CH_3)_2$ $R_1$=4—$OC_5H_{11}$)
m.p. 157—159°C
I.R. (nujol) ν ($cm^{-1}$): 1635 (CO)
H—NMR(DMSO) δ (p.p.m.): 0.95 (t, $CH_3$) 1.0 (d, 2×$CH_3$) 1.2—2.0 (m, 3×$CH_2$) 2.6—3.0 (m, $CH_2$+CH) 3.8—4.3 (m, $CH_2$+CH) 6.9—8.05 (m, 2×$C_6H_4$) 10.0 (s, NH)

9) 1[4(p-hexoxybenzamido)phenoxy]-3-isopropylamino-2-propanol hydrochloride
(Form. I R—$CH(CH_3)_2HCl$ $R_1$=4—$OC_6H_{13}$)
m.p. 199—201°C
I.R. (nujol) ν ($cm^{-1}$): 1635 (CO)
H—NMR(DMSO) δ (p.p.m.): 0.8 (t, $CH_3$) 1.10 (d, 2×$CH_3$) 1.3—1.9 (m, 4×$CH_2$) 2.9—3.6 (m, $CH_2$+CH)

10) 1-isopropylamino-3-[4(p-octoxybenzamido)phenoxy]-2-propanol
(Form. I R=$CH(CH_3)_2$ $R_1$=4—$OC_8H_{17}$)
m.p. 116—118°C
I.R. (nujol) ν ($cm^{-1}$): 1645 (CO)

11) 1[4(p-benzyloxybenzamido)phenoxy]-3-isopropylamino-2-propanol hydrochloride
(Form. I R=$CH(CH_3)_2HCl$ $R_1$=4—$OCH_2$—$C_6H_5$)
m.p. 217—220°C
I.R. (nujol) ν ($cm^{-1}$): 1645 (CO)
H—NMR(DMSO) δ (p.p.m.): 1.3 (d, 2×$CH_3$) 2.8—3.2 (m, $CH_2$+CH) 3.8—4.4 (m, $CH_2$+CH) 5.2 (s, $CH_2$) 6.85—7.10 (m, 2×$C_6H_4$+$C_6H_5$)

Examples of pharmaceutically acceptable non-toxic salts of the compounds above mentioned are: chloride, bromide, iodide, phosphate, sulfate, tartrate, citrate, as well as methyliodide, methylbromide, ethylbromide, ethyliodide. The optical isomers of the compounds of general formula I are also included in the present invention.

Another object of the present invention is to provide a process for preparing the derivatives of general formula I by starting from a compound of general formula II

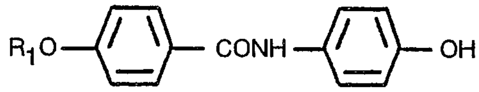

II

e.g. the following compounds

| $R_1$ | CONH position | m.p. (°C) |
|---|---|---|
| 4-$CH_3$ | 4 | 236—8 |
| 4-$C_2H_5$ | 4 | 235—7 |
| 4-$C_3H_7$ | 4 | 223—5 |
| 4-iso$C_3H_7$ | 4 | 213—5 |
| 4-$C_4H_9$ | 4 | 217—20 |
| 4-$C_5H_{11}$ | 4 | 216—17 |
| 4-$C_6H_{13}$ | 4 | 205—7 |
| 4-$C_6H_5CH_2$ | 4 | 242—44 |

or by starting from a compound of formula:

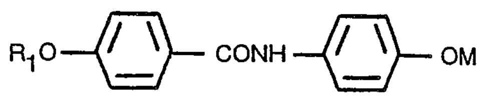

IIa

where $R_1$ has the same meaning as in formula I, and M is an alkali metal and reacting it with a compound of formula:

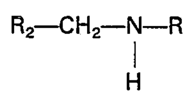

III

where R has the same meaning as in formula I, $R_2$ is a group

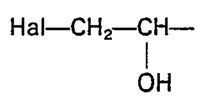

or a group

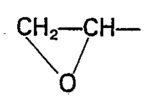

where Hal is a halogen atom. The reaction is preferably carried out in a solvent such as alcohol, dioxane, dimethylformamide, water.

The compounds of general formula II can be obtained, for example, by starting from the chloride of the appropriate benzoic acid and aminophenol.

The compounds of formula III can be prepared for example by starting from a 1,3-dihalogen-2-propanol or from epihalogenhydrin with an amine having formula

$RNH_2$

where R has the same meaning as in formula I.

The compounds of general formula I can also be prepared by reacting a compound of formula

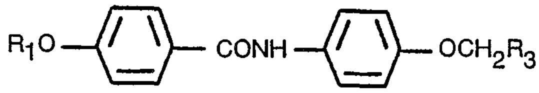 IV

where $R_1$ has the same meaning as in formula I and $R_3$ is a group

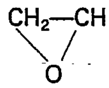 or a group 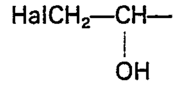

where Hal is a halogen atom, with an amine having formula: $NH_2R$ where R is defined as in formula I. In this reaction the solvent to be used is either an amine excess or a preferably polar solvent, as for example ethanol.

The halogenhydrin of formula IV can be obtained by reacting a compound of formula II with epihalogenhydrin as for example epichlorohydrin. The epoxide of formula IV can be obtained through conventional methods, for example, by dehydrohalogenation of the already cited halogenhydrin with sodium hydroxide solution.

The compounds of formula I can also be obtained by the reduction of a compound having formula

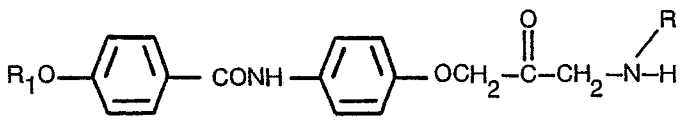 V

where R and $R_1$ are defined as in formula I.

The reduction of the compound V is preferably carried out with hydride, as for example sodium boron hydride.

The compounds of formula V can be obtained by starting from compounds of formula II with 1,3 dihalogenacetone, as for example 1,3-dichloroacetone, through the corresponding compound

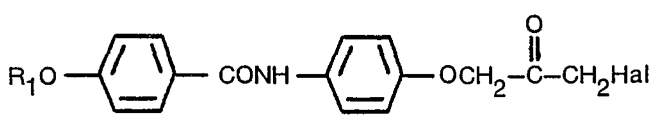 VI

and subsequent treatment with amine $RNH_2$ where R is defined as in formula I.

The compound in racemic form can be reacted with an optically active acid, followed by fractionated crystallization of the diastereoisomers mixture obtained with a suitable solvent, such as for example ethanol, and then the optically active derivative is freed from the salt by treatment with a base.

The invention has also the object to provide pharmaceutical compositions including, as active ingredient, one or more derivatives according to the general formula I (or addition salts) in association with a pharmaceutically acceptable diluent or carrier.

As appropriate compositions, there may be mentioned for instance, tablets, capsules, aqueous or oily solutions or suspensions, emulsions, or dispersing powders, pulverizing or aerosol compositions.

The invention is illustrated by the following examples:

### Example 1

1-isopropylamino-3[4(p-methoxybenzamido)phenoxy]-2-propanol

0.02 mol of 4(p-methoxybenzamido)phenol are put into 120 ml of a 0.02N potassium hydroxide aqueous solution, then 0.045 mol of epichlorohydrin are added in 10 ml methanol and the mixture is kept stirring at room temperature for two days.

The reaction product is filtered out, washed twice with water (50 ml), then the resulting crude epoxide is suspended in methanol (300 ml), to which water (0.5 ml) and isopropylamine (0.6 mol) are added. After stirring for two days at room temperature, the reaction product is filtered out, vacuum dried, and added to some water again.

The reaction product, once crystallized, yields 1-isopropylamino-3-[4(p-methoxybenzamido)phenoxy]-2-propanol, m.p. 174—176°C.

This product, dissolved in ether over anhydrous gaseous hydrochloric acid yields the hydrochloride (ethyl alcohol) m.p. 208—210°C.

Example 2

1-tert.-butylamino-3[4(p-methoxybenzamido)phenoxy]-2-propanol

To 0.028 mol of crude 1-[4(p-methoxybenzamido)phenoxy]-2,3-epoxypropane, obtained as in example 1, and suspended in methanol (300 ml), water (0.5 ml) and tert.-butylamine (0.6 mol) are added. After stirring for 48 h at room temperature, the reaction product is filtered out, vacuum dried, taken up with acetone (20 ml) and subjected to gaseous hydrochloric acid: the precipitate thus obtained is filtered out and dissolved in water, and by alkalization there is obtained a precipitate which is filtered, washed and dried: the latter, once dissolved in acetone and subjected to gaseous HCl, yields a hydrochloride(dioxane/isopropanol) (m.p. 210—212°C).

Example 3

1[4(p-butoxybenzamido)phenoxy]-3-isopropylamino-2-propanol

0.08 mol of 4(p-butoxybenzamido)phenol are heated for 6 h on water bath in epichlorohydrin (0.64 mol) and piperidine (0.1 ml). The epichlorohydrin excess is distilled, then the residue is taken up with ether and filtered.

The crude product 1[4(p-butoxybenzamido)phenoxy]-3-chloro-2-propanol thus obtained is suspended in methanol (600 ml); after addition of isopropylamine (1.8 mol), the product is stirred at room temperature for 48 hours and then filtered out. The filtrate is dried, taken up with acetone (15 ml), filtered and crystallized from ethanol: m.p. 152—154°C.

Example 4

1[4(p-allyloxybenzamido)phenoxy]-3-isopropylamino-2-propanol

0.08 mol of 4(p-allyloxybenzamido)phenol are heated for 6 h on water bath in epichlorohydrin (0.64 mol) and piperidine (0.1 ml). The epichlorohydrin excess is distilled, then the residue is taken up with ether and filtered.

The crude product 1[4(p-allyloxybenzamido)phenoxy]-3-chloro-2-propanol thus obtained is suspended in methanol (1 lt) to which isopropylamine (1.8 mol) is added. After stirring for 72 h at room temperature, the product is filtered, dried, washed with water, filtered and dried again, and then the product is taken up with acetone and subjected to anhydrous gaseous hydrochloric acid to obtain the hydrochloride crystallized from isopropanol (m.p. 200—202°C).

Example 5

1[4(p-ethoxybenzamido)phenoxy]-3-isopropylamino-2-propanol

0.02 mol of 4(p-ethoxybenzamido)phenol are put into 120 ml of potassium hydroxide 0.02N acqueous solution, afterwards epichlorohydrin (0.045 mol) in methanol (10 ml) is added, followed by stirring at room temperature for 48 h. The product is filtered, washed twice with water (50 ml) and the crude 1[4(p-ethoxybenzamido)phenoxy]-2,3-epoxypropane is suspended in methanol (200 ml), and water (0.2 ml) and isopropylamine (0.5 mol) are added.

After having stirred for two days at room temperature, the product is filtered and dried. It is dissolved in acetone and treated with anhydrous gaseous hydrochloric acid: the hydrochloride crystallized from isopropanol has m.p. of 215—217°C.

Biological activity

The beta-sympatholytic activity of the compounds under examination has been tested in *vivo* as antagonist to the $\beta_1$-chronotropic effects (cardiac frequency increase), $\beta_1$-inotropic effects (systolic ejection velocity increase), and $\beta_2$ effects (diastolic pressure lowering) of the isoprenaline upon the anaesthetized and reserpine pretreated rat, after oral administration, and in *vitro* as antagonist to the $\beta_1$ chronotropic effects (cavy right atrium), $\beta_1$ inotropic effects (cavy ventricle) and $\beta_2$ effects (cavy trachea) of the isoprenaline.

From in vivo and in vitro results it is evident that the 1-alkylamino-3[4(p-alkoxybenzamido)phenoxy]-2-propanol derivatives, which are the object of the present invention, show β-blocking activity of a competitive type and, on the contrary of β-blocking constituents as propranolol, they show cardioselectivity and inherent sympathicomimetic activity, the latter being revealed by the relevant increase of the cardiac frequency in the reserpine pretreated animal at rest.

The beta-sympatholytic activity of the 1-alkylamino-3[4(p-alkoxybenzamido)phenoxy]-2-propanol compounds is in vivo significantly greater than the compounds such as propranolol which, although more active in vitro, are subjected to a relevant decrease of the in *vivo* activity owing to an intense liver metabolism. All these favourable characteristics, that is, cardioselectivity, inherent sympathicomimetic activity and lack of a significant power gap between vivo and vitro are also present in the 4-benzamido (or acetamido)phenoxy-3-isopropylamine-2-propanol derivatives, such as for example 1-(4-benzamido phenoxy)-3-isopropylamino-2-propanol (Ralph Howe, Leslie Harold Smith U.S. 3.408.387; A. F. Crowther, R. Howe, L. H. Smith, J. Med. Chem. *14* (6), 511, 1971), 1[4(-p-chlorobenzamido)phenoxy]-3-isopropylamino-2-propanol (A. F. Crowther, R. Howe, L. M. Smith, J. Med. Chem. *14*, (6), 511, 1971), practolol (Howe, Smith, U.S. 3.408.387; Scales, Cosgrove, J. Pharmacol. Exp. Ther. *175*, 338, 1970). On the other hand, compared to the latter compounds, the 1[4(p-alkoxybenzamido)phenoxy]-3-isopropylamino-2-propanol

derivatives present also an important pharmacological characteristic, namely a significantly greater power to block the $\beta_1$ chronotropic effects with respect to the $\beta_1$ inotropic effects of the isoprenaline. The in vivo tests have shown that at certain dosage levels the compounds of this invention determine a significant beta-sympatholytic effect on the cardiac frequency chronotropism) without a concurrent reduction of the myocardium contractility (inotropism).

Since the antianginal activity of the beta-sympatholytic agents is performed by the reduction of the cardiac frequency (Bardaux A. et Eur. J. Pharmacol. *39,* 287, 1966; Gross G. J. and Waltier Dc. J. Pharm. Exp. Ther. *203,* 544, 1977), the 1[4(p-alkoxybenzamido)phenoxy]-3-isopropylamino-2-propanol derivatives should carry out, as compared to the beta-blocking agents of current use in therapy, a significant anti-ischemic action with a very minor risk of determining a hypokinetic condition of the myocardium which, in turn, is considered a decisive element for the appearance of angina crisis (Kaverina N. V. and Chumburidze V. B. Pharmac. Ther. *4,* 109, 1979).

It is also interesting to note that the 1[4(p-alkyloxybenzamido . . . or 1[4(m-alkoxybenzamido)phenoxy]-3-isopropylamin-2-propanol derivatives, as well as the 1[2(p-alkoxybenzamido . . . or 1[3(p-alkoxybenzamido)phenoxy]-3-isopropylamino-2-propanol derivatives, although endowed with β-sympatholytic activity of a competitive type, with cardioselectivity, and with inherent sympathicomimetic activity, present a minor activity as compared to the corresponding derivatives of the present invention, and in some cases they are lacking in chronotropic activity.

From these results it will be apparent the fundamental importance of the full replacement of the phenoxy group with a p-alkoxybenzamido group.

The replacement of an alkoxy group with another group will determine either a distinct reduction of activity or a loss of selectivity: a reduction of activity or a loss of selectivity will occur also by introducing more alkoxy groups.

**Claims for the Contracting States: BE CH DE FR GB LI LU NL SE**

1. A derivative of 1-alkylamino-3-[4(p-alkoxybenzamido)phenoxy]-2-propanol of general formula I:

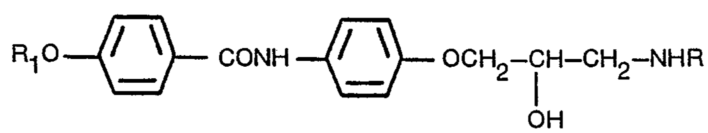

where $R_1$ is a linear or branched alkyl chain with 1 to 8 carbon atoms included, an alkenyl chain with 2 to 8 carbon atoms included, or an aralkyl chain with 7 to 10 carbon atoms included: R is a linear or branched alkyl group with 1 to 8 carbon atoms included.

2. A compound as claimed in claim 1, in optically active form.

3. Pharmaceutically acceptable, non toxic salts of compounds according to claim 1.

4. Pharmaceutically acceptable, non toxic salts of compounds according to claim 2.

5. A compound according to claim 1, selected from the group consisting of 1-isopropylamino-3[4(p-methoxybenzamido)phenoxy]-2-propanol and a pharmaceutically acceptable salt thereof, 1-t-butylamino-3-[4(p-methoxybenzamido)phenoxy]-2-propanol and a pharmaceutically acceptable salt thereof, 1-[4(p-ethoxybenzamido)phenoxy]-3-isopropylamino-2-propanol and a pharmaceutically acceptable salt thereof, 1-[4(p-allyloxybenzamido)phenoxy]-3-isopropylamino-2-propanol and a pharmaceutically acceptable salt thereof, 1-isopropylamino-3-[4(p-propoxybenzamido)phenoxy]-2-propanol and a pharmaceutically acceptable salt thereof, 1-isopropylamino-3-[4(p-isopropoxybenzamido)phenoxy]-2-propanol and a pharmaceutically acceptable salt thereof, 1-[4(p-butoxybenzamido)phenoxy]-3-isopropylamino-2-propanol and a pharmaceutically acceptable salt thereof, 1-[4(p-butoxybenzamido)phenoxy]-3-isopropylamino-2-propanol and a pharmaceutically acceptable salt thereof, 1-isopropylamino-3-[4(p-pentoxybenzamido)phenoxy]-2-propanol and a pharmaceutically acceptable salt thereof, 1-[4(p-hexoxybenzamido)phenoxy]-3-isopropylamino-2-propanol and a pharmaceutically acceptable salt thereof, 1-isopropylamino-3-[4(p-octoxybenzamido)phenoxy]-2-propanol and a pharmaceutically acceptable salt thereof, 1-[4(p-benzyloxybenzamido)phenoxy]-3-isopropylamino-2-propanol and a pharmaceutically acceptable salt thereof.

6. Process of synthesis for obtaining the compounds according to claim 1, including the reaction between the suitable benzamidophenol, in alkaline ambient, and the appropriate 3-amino-2-hydroxy-1-halogen propane or the appropriate 3-amino-1,2-epoxypropane.

7. Process of synthesis for obtaining the compounds according to claim 1, including the reaction of the suitable benzamidophenoxy epihalogenhydrin (or epoxide) with the appropriate amine.

8. Process of synthesis for obtaining the compounds according to claim 1, including the reaction starting from the suitable benzamidophenol with a dihalogen acetone, subsequent treatment with the appropriate amine and then reduction to the corresponding alcohol.

9. A pharmaceutical composition comprising at least a compound according to claim 1, as active principle in association with a carrier.

10. A composition according to claim 9, in a suitable form for oral, parenteral or rectal administration.

**Claims for the Contracting State: AT**

1. Process for preparing derivatives of 1-alkylamino-3-4[(p-alkoxybenzamido)phenoxy]-2-propanol of general formula I

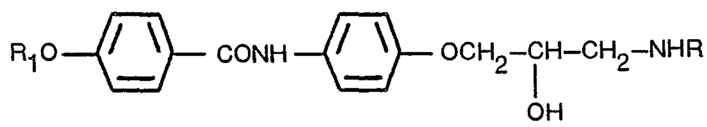

I

where $R_1$ is a linear or branched alkyl chain with 1 to 8 carbon atoms included, an alkenyl chain with 2 to 8 carbon atoms included, or an aralkyl chain with 7 to 10 carbon atoms included, R is a linear or branched alkyl group with 1 to 8 carbon atoms included, characterized in that a benzamidophenol of general formula II

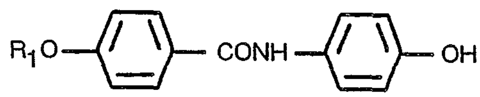

II

is reacted with a compound of formula III

$R_2$—$CH_2$—NH—R III

where R has the same meaning as in formula I and $R_2$ is a group

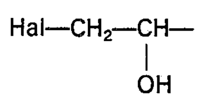

or a group

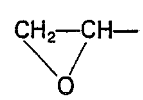

where Hal is a halogen atom, or that a compound of formula IIa

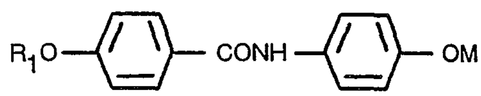

IIa

where $R_1$ has the same meaning as in formula I and M is an alkali metal is reacted with a compound of formula III and that optionally pharmaceutically acceptable salts are prepared therefrom.

2. Process for preparing compounds of general formula I characterized in that a compound of formula IV

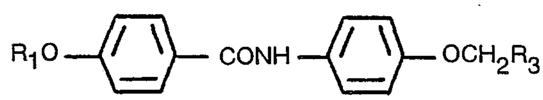

IV

where $R_1$ has the same meaning as in formula I and $R_3$ is a group

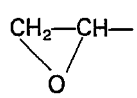

or

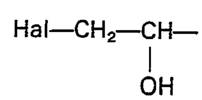

is reacted with an amine $NH_2R$ where R is defined as in formula I.

3. Process for preparing compounds of formula I characterized in that starting from a compound of formula II with 1,3-dihalogenacetone the compound VI so obtained

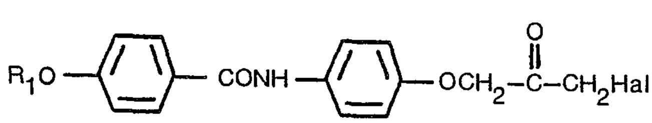

VI

is treated with an amine $RNH_2$ where R is defined as in formula I and that thereafter the resulting compound of formula V

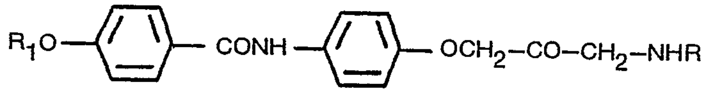

V

is reduced.

4. Process according to claim 1 to 3 characterised in that the process product is selected from the group consisting of 1-isopropylamino-3[4(p-methoxybenzamido)phenoxy]-2-propanol and a pharmaceutically salt thereof, 1-t-butylamino-3-[4(p-methoxybenzamido)phenoxy]-2-propanol and a pharmaceutically acceptable salt thereof, 1-[4(p-ethoxybenzamido)phenoxy]-3-isopropylamino-2-propanol and a pharmaceutically acceptable salt thereof, 1-[4(p-allyloxybenzamido)phenoxy]-3-isopropylamino-2-propanol and a pharmaceutically acceptable salt thereof, 1-isopropylamino-3-[4(p-propoxybenzamido)phenoxy]-2-propanol and a pharmaceutically acceptable salt thereof, 1-isopropylamino-3-[4(p-isopropoxybenzamido)phenoxy]-2-propanol and a pharmaceutically acceptable salt thereof, 1-[4(p-butoxybenzamido)phenoxy]-3-isopropylamino-2-propanol and a pharmaceutically acceptable salt thereof, 1-isopropylamino-3-[4(p-pentoxybenzamido)phenoxy]-2-propanol and a pharmaceutically acceptable salt thereof, 1-[4(p-hexoxybenzamido)phenoxy]-3-isopropylamino-2-propanol and a pharmaceutically acceptable salt thereof, 1-isopropylamino-3-[4(p-octoxybenzamido)phenoxy]-2-propanol and a pharmaceutically acceptable salt thereof, 1-[4(p-benzyloxybenzamido)phenoxy]-3-isopropylamino-2-propanol and a pharmaceutically acceptable salt thereof.

**Patentansprüche für die Vertragsstaaten: BE CH DE FR GB LI LU NL SE**

1. 1-Alkylamino-3-[4(p-alkoxybenzamido)phenoxy]-2-propanolderivate der allgemeinen Formel I:

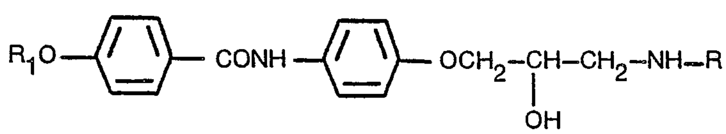

in der $R_1$ eine lineare oder verzweigte Alkylgruppe mit 1 bis einschließlich 8 Kohlenstoffatomen, eine Alkenylgruppe mit 2 bis einschließlich 8 Kohlenstoffatomen oder eine Aralkylgruppe mit 7 bis einschließlich 10 Kohlenstoffatomen und R eine lineare oder verzweigte Alkylgruppe mit 1 bis einschließlich 8 Kohlenstoffatomen bedeuten.

2. Verbindung nach Anspruch 1 in ihrer optisch aktiven Form.

3. Pharmazeutisch verträgliche, nicht-toxische Salze der Verbindungen nach Anspruch 1.

4. Pharmazeutisch verträgliche, nicht-toxische Salze der Verbindungen nach Anspruch 2.

5. Verbindung nach Anspruch 1, ausgewählt aus der Gruppe der folgenden Verbindungen: 1-Isopropylamino-3-[4(p-methoxybenzamido)phenoxy]-2-propanol und ein pharmazeutisch verträgliches Salz desselben, 1-t-Butylamino-3-[4(p-methoxybenzamido)phenoxy]-2-propanol und ein pharmazeutisch verträgliches Salz desselben, 1-[4(p-Ethoxybenzamido)phenoxy]-3-isopropylamino-2-propanol und ein pharmazeutisch verträgliches Salz desselben, 1-[4(p-Allyloxybenzamido)phenoxy]-3-isopropylamino-2-propanol und ein pharmazeutisch verträgliches Salz desselben, 1-Isopropylamino-3-[4(p-propoxybenzamido)phenoxy]-2-propanol und ein pharmazeutisch verträgliches Salz desselben, 1-Isopropylamino-3-[4(p-isopropoxybenzamido)phenoxy]-2-propanol und ein pharmazeutisch verträgliches Salz desselben, 1-[4(p-Butoxybenzamido)phenoxy]-3-isopropylamino-2-propanol und ein pharmazeutisch verträgliches Salz desselben, 1-isopropylamino-3-[4(p-Pentoxybenzamido)phenoxy]-2-propanol und ein pharmazeutisch verträgliches Salz desselben, 1-[4(p-Hexoxybenzamido)-phenoxy]-3-isopropylamino-2-propanol und ein pharmazeutisch verträgliches Salz desselben, 1-Isopropylamino-3-[4(p-octoxybenzamido)phenoxy]-2-propanol und ein pharmazeutisch verträgliches Salz desselben, 1-[4(p-Benzyloxybenzamido)phenoxy]-3-isopropylamino-2-propanol und ein pharmazeutisch verträgliches Salz desselben.

6. Verfahren zur Herstellung der Verbindungen nach Anspruch 1, gekennzeichnet durch eine Reaktion zwischen einem geeigneten Benzamidophenol und einem geeigneten 3-Amino-2-hydroxy-1-halogenpropan oder einem geeigneten 3-Amino-1,2-epoxypropan in einem alkalischen Milieu.

7. Verfahren zur Herstellung der Verbindungen nach Anspruch 1, gekennzeichnet durch eine Umsetzung eines geeigneten Benzamidophenoxy-epihalogenhydrins (oder -epoxids) mit einem geeigneten Amin.

8. Verfahren zur Herstellung der Verbindungen nach Anspruch 1, gekennzeichnet durch eine von einem geeigneten Benzamidophenol ausgehende Umsetzung mit einem Dihalogenaceton, gefolgt von einer Behandlung mit einem geeigneten Amin und anschließend einer Reduktion zu dem entsprechenden Alkohol.

9. Pharmazeutische Zusammensetzung, enthaltend mindestens eine Verbindung nach Anspruch 1 als Wirkstoff zusammen mit einem Träger.

10. Zusammensetzung nach Anspruch 9 in einer für die orale, parenterale oder rektale Verabreichung geeigneten Formulierung.

**Patentansprüche für den Vertragsstaat: AT**

1. Verfahren zur Herstellung von 1-Alkylamino-3-[4(p-alkoxybenzamido)phenoxy]-2-propanolderivaten der allgemeinen Formel I

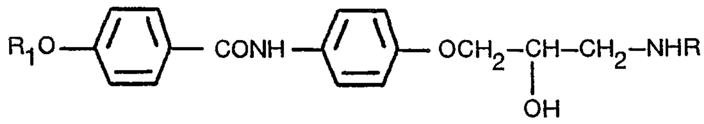
I

in der $R_1$ eine lineare oder verzweigte Alkylgruppe mit 1 bis einschließlich 8 Kohlenstoffatomen, eine Alkenylgruppe mit 2 bis einschließlich 8 Kohlenstoffatomen oder eine Aralkylgruppe mit 7 bis einschließlich 10 Kohlenstoffatomen und R eine lineare oder verzweigte Alkylgruppe mit 1 bis einschließlich 8 Kohlenstoffatomen bedeuten, dadurch gekennzeichnet, daß man ein Benzamidophenol der allgemeinen Formel II

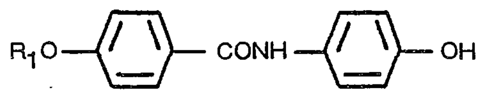
II

mit einer Verbindung der Formel III

$$R_2—CH_2—NH—R \qquad III$$

wobei R wie für Formel I definiert ist und $R_2$ eine Gruppe

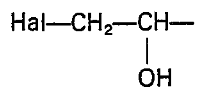
oder eine Gruppe
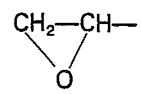

ist und Hal ein Halogenatom bedeutet, oder daß man eine Verbindung der Formel IIa

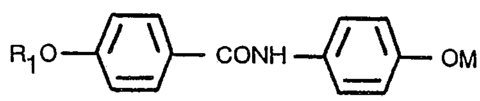
IIa

in der $R_1$ die für die Formel I angegebene Bedeutung aufweist und M ein Alkalimetall ist, mit einer Verbindung der Formel III umsetzt und daraus gegebenenfalls pharmazeutisch verträgliche Salze herstellt.

2. Verfahren zur Herstellung der Verbindungen der allgemeinen Formel I, dadurch gekennzeichnet, daß man eine Verbindung der Formel IV

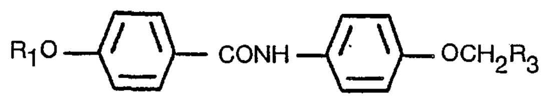
IV

in der $R_1$ wie für Formel I definiert ist und $R_3$ eine Gruppe

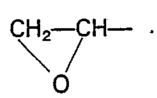
oder
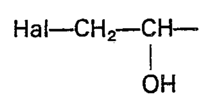

bedeuten, mit einem Amin $NH_2R$ umsetzt, wobei R wie für Formel I definiert ist.

3. Verfahren zur Herstellung der Verbindungen der Formel I, dadurch gekennzeichnet, daß man ausgehend von einer Verbindung der Formel II und 1,3-Dihalogenaceton die so erhaltene Verbindung VI

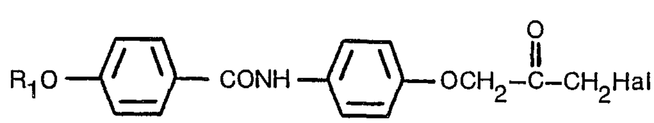
VI

mit einem Amin $RNH_2$ umsetzt, wobei R wie für Formel I definiert ist, und danach die erhaltene Verbindung der Formel V

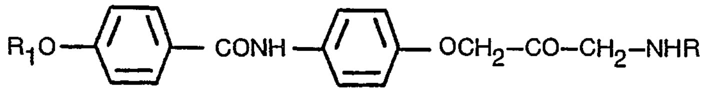

V

reduziert.

4. Verfahren nach einem der Ansprüche 1 bis 3, dadurch gekennzeichnet, daß man als Verfahrensprodukt eine Verbindung herstellt, die aus der folgenden Gruppe ausgewählt ist: 1-Isopropylamino-3-[4(p-methoxybenzamido)phenoxy]-2-propanol und ein pharmazeutisch verträgliches Salz desselben, 1-t-Butylamino-3-[4(p-methoxybenzamido)phenoxy]-2-propanol und ein pharmazeutisch verträgliches Salz desselben, 1-[4(p-Ethoxybenzamido)phenoxy]-3-isopropylamino-2-propanol und ein pharmazeutisch verträgliches Salz desselben, 1-[4(p-Allyloxybenzamido)phenoxy]-3-isopropylamino-2-propanol und ein pharmazeutisch verträgliches Salz desselben, 1-Isopropylamino-3-[4(p-propoxybenzamido)phenoxy]-2-propanol und ein pharmazeutisch verträgliches Salz desselben, 1-Isopropylamino-3-[4(p-isopropoxybenzamido)phenoxy]-2-propanol und ein pharmazeutisch verträgliches Salz desselben, 1-[4(p-Butoxybenzamido)phenoxy]-3-isopropylamino-2-propanol und ein pharmazeutisch verträgliches Salz desselben, 1-isopropylamino-3-[4(p-Pentoxybenzamido)phenoxy]-2-propanol und ein pharmazeutisch verträgliches Salz desselben, 1-[4(p-Hexoxybenzamido)-phenoxy]-3-isopropylamino-2-propanol und ein pharmazeutisch verträgliches Salz desselben, 1-Isopropylamino-3-[4(p-octoxybenzamido)phenoxy]-2-propanol und ein pharmazeutisch verträgliches Salz desselben, 1-[4(p-Benzyloxybenzamido)phenoxy]-3-isopropylamino-2-propanol und ein pharmazeutisch verträgliches Salz desselben.

**Revendications pour les Etats contractants: BE CH DE FR GB LI LU NL SE**

1. Dérivé de 1-alcoylamino-3-[4(p-alcoxybenzamido)phénoxy]-2-propanol de formule générale I:

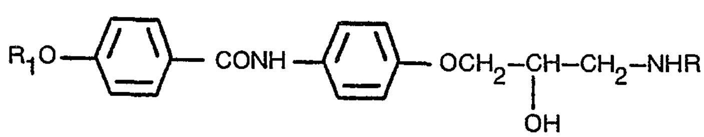

dans laquelle $R_1$ est une chaîne alcoyle linéaire ou ramifiée avec 1 à 8 (inclus) atomes de carbone, une chaîne alcényle avec 2 à 8 (inclus) atomes de carbone, ou une chaîne alcoylaryle avec 7 à 10 (inclus) atomes de carbone; R est un groupe alcoyle linéaire ou ramifié avec 1 à 8 (inclus) atomes de carbone.

2. Composé selon la revendication 1, sous forme optiquement active.

3. Sels non toxiques, pharmaceutiquement acceptables, de composés selon la revendication 1.

4. Sels non toxiques, pharmaceutiquement acceptables, de composés selon la revendication 2.

5. Composé selon la revendication 1, choisi dans le groupe comprenant le 1-isopropylamino-3-[4(p-méthoxybenzamido)phénoxy]-2-propanol et un sel pharmaceutiquement acceptable, le 1-t-butylamino-3-[4(p-méthoxybenzamido)phénoxy]-2-propanol et un sel pharmaceutiquement acceptable, le 1-[4(p-éthoxybenzamido)phénoxy]-3-isopropylamino-2-propanol et un sel pharmaceutiquement acceptable, le 1-[4(p-allyloxybenzamido)phénoxy]-3-isopropylamino-2-propanol et un sel pharmaceutiquement acceptable, le 1-isopropylamino-3-[4(p-propoxybenzamido)phénoxy]-2-propanol et un sel pharmaceutiquement acceptable, le 1-isopropylamino-3-[4(p-isopropoxybenzamido)phénoxy]-2-propanol et un sel pharmaceutiquement acceptable, le 1-[4(p-butoxybenzamido)phénoxy]-3-isopropylamino-2-propanol et un sel pharmaceutiquement acceptable, le 1-isopropylamino-3-[4(p-pentoxybenzamido)phénoxy]-2-propanol et un sel pharmaceutiquement acceptable, le 1-[4(p-hexoxybenzamido)phénoxy]-3-isopropylamino-2-propanol et un sel pharmaceutiquement acceptable, le 1-isopropylamino-3-[4(p-octoxybenzamido)phénoxy]-2-propanol et un sel pharmaceutiquement acceptable, le 1-[4(p-benzyloxybenzamido)phénoxy]-3-isopropylamino-2-propanol et un sel pharmaceutiquement acceptable.

6. Procédé de synthèse pour obtenir les composés selon la revendication 1, comprenant la réaction entre le benzamidophénol approprié, en milieu alcalin, et le 3-amino-2-hydroxy-1-halogéno-propane approprié ou le 3-amino-1,2-époxypropane approprié.

7. Procédé de synthèse pour obtenir les composés selon la revendication 1, comprenant la réaction de la benzamidophénoxy-épihalogénhydrine (ou de l'époxyde) appropriée avec l'amine appropriée.

8. Procédé de synthèse pour obtenir les composés selon la revendication 1, comprenant la réaction à partir du benzamidophénol approprié avec une dihalogénacétone, le traitement subséquent avec l'amine appropriée, puis la réduction en l'alcool correspondant.

9. Composition pharmaceutique comprenant au moins un composé selon la revendication 1, comme principe actif, en association avec un support.

10. Composition selon la revendication 9, sous une forme appropriée pour l'administration orale, parentérale ou rectale.

**Revendications pour l'Etat contractant: AT**

1. Procédé de synthèse pour obtenir un dérivé de 1-alcoylamino-3-[4(p-alcoxybenzamido)phénoxy]-2-propanol de formule générale I:

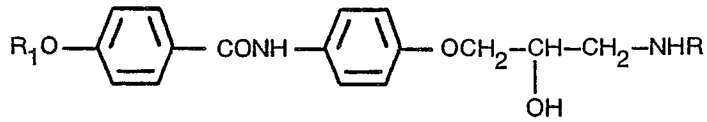

I

dans laquelle $R_1$ est une chaîne alcoyle linéaire ou ramifiée avec 1 à 8 (inclus) atomes de carbone, une chaîne alcényle avec 2 à 8 (inclus) atomes de carbone, ou une chaîne alcoylaryle avec 7 à 10 (inclus) atomes de carbone; R est un groupe alcoyle linéaire ou ramifié avec 1 à 8 (inclus) atomes de carbone, comprenant la réaction d'un benzamidophénol de formule générale II

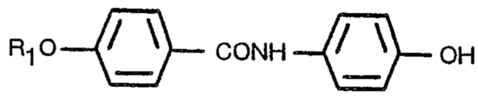

II

avec un composé de formule générale III

$$R_2—CH_2—NH—R \qquad III$$

dans laquelle R est défini comme en formule I, $R_2$ est un groupe

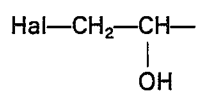

ou un groupe

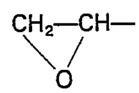

et Hal est un atom d'halogéne, ou la réaction d'un composé de formule générale IIa

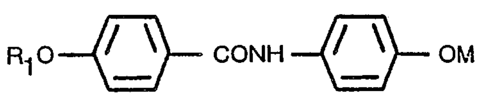

IIa

dans laquelle $R_1$ est défini comme en formule I et M est un métal d'alkali, avec le composé de formule III et, le cas échéant, la réction du composé obtenu pour préparer de sels pharmaceutiquement acceptables.

2. Procédé de synthèse pour obtenir les composés de formule générale I, comprenant la réaction d'un composé de formule générale IV

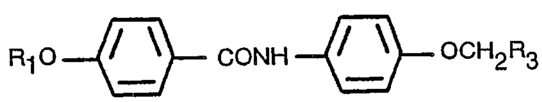

IV

dans laquelle $R_1$ est défini comme en formule I et $R_3$ est un groupe

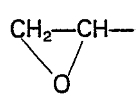

ou

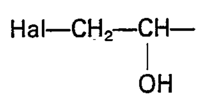

avec un amine $NH_2R$, où R est défini comme en formule I.

3. Procédé de synthèse pour obtenir les composés de formule générale I, comprenant la réaction à partir d'un composé de formule générale II et d'un 1,3-dihalogénacétone et le traitement subsequent du composé obtenu de formule VI

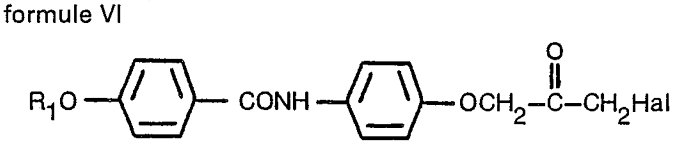

VI

avec un amine $RNH_2$, où R est défini comme en formule I, et puis la réduction du composé obtenu de formule V

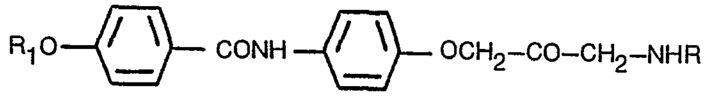

V

4. Procédé de synthèse selon une des revendications 1 à 3 pour la préparation d'un composé choisi dans le groupe comprenant le 1-isopropylamino-3-[4(p-méthoxybenzamido)phénoxy]-2-propanol et un sel pharmaceutiquement acceptable, le 1-t-butylamino-3-[4(p-méthoxybenzamido)phénoxy]-2-propanol et un sel pharmaceutiquement acceptable, le 1-[4(p-éthoxybenzamido)phénoxy]-3-isopropylamino-2-propanol et un sel pharmaceutiquement acceptable, le 1-[4(p-allyloxybenzamido)phénoxy]-3-isopropylamino-2-propanol et un sel pharmaceutiquement acceptable, le 1-isopropylamino-3-[4(p-propoxybenzamido)phénoxy]-2-propanol et un sel pharmaceutiquement acceptable, le 1-isopropylamino-3-[4(p-isopropoxybenzamido)phénoxy]-2-propanol et un sel pharmaceutiquement acceptable, le 1-[4(p-butoxybenzamido)phénoxy]-3-isopropylamino-2-propanol et un sel pharmaceutiquement acceptable, le 1-isopropylamino-3-[4(p-pentoxybenzamido)phénoxy]-2-propanol et un sel pharmaceutiquement acceptable, le 1-[4(p-hexoxybenzamido)phénoxy]-3-isopropylamino-2-propanol et un sel pharmaceutiquement acceptable, le 1-isopropylamino-3-[4(p-octoxybenzamido)phénoxy]-2-propanol et un sel pharmaceutiquement acceptable, le 1-[4(p-benzyloxybenzamido)phénoxy]-3-isopropylamino-2-propanol et un sel pharmaceutiquement acceptable.